(19) Europäisches Patentamt
European Patent Office
Office européen des brevets

(11) Veröffentlichungsnummer : **0 103 173**
**B1**

# (12) EUROPÄISCHE PATENTSCHRIFT

(45) Veröffentlichungstag der Patentschrift : **29.07.87**

(51) Int. Cl.⁴ : **C 07 D319/08**, A 01 N 47/22

(21) Anmeldenummer : **83107911.6**

(22) Anmeldetag : **10.08.83**

(54) **Carbaminsäureester, Verfahren und Ausgangsmaterialien zu deren Herstellung, Schädlingsbekämpfungsmittel, die diese Carbaminsäureester als Wirkstoffe enthalten, sowie Verwendung solcher Carbaminsäureester und Mittel zur Bekämpfung von Schädlingen.**

(30) Priorität : **11.08.82 CH 4812/82**
**03.06.83 CH 3044/83**

(43) Veröffentlichungstag der Anmeldung : **21.03.84 Patentblatt 84/12**

(45) Bekanntmachung des Hinweises auf die Patenterteilung : **29.07.87 Patentblatt 87/31**

(84) Benannte Vertragsstaaten : **AT BE CH DE FR GB IT LI LU NL SE**

(56) Entgegenhaltungen :
**FR-A- 2 359 136**
**RESEARCH DISCLOSURE, Nr. 179, März 1979, Seite 130, Nr. 17952 "Nematicide"**
**The Merck Index, 9. Ausgabe (1976), Seite 1036**
**K.H. Büchel, Pflanzenschutz und Schädlingsbekämpfung, Thieme Verlag, Stuttgart, 1977, Seite 65**
**Die Akte enthält technische Angaben, die nach dem Eingang der Anmeldung eingereicht wurden und die *nicht in dieser Patentschrift enthalten sind.***

(73) Patentinhaber : **F. HOFFMANN-LA ROCHE & CO. Aktiengesellschaft**

**CH-4002 Basel (CH)**

(72) Erfinder : **Zurflüh, René, Dr.**
**Dachslenbergstrasse 54**
**CH-8180 Bülach (CH)**

(74) Vertreter : **Lederer, Franz, Dr. et al**
**Vanderwerth, Lederer & Riederer Patentanwälte Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 103 173 B1**

Jouve, 18, rue St-Denis, 75001 Paris, France

**Beschreibung**

Die Erfindung betrifft Carbaminsäureester der allgemeinen Formel

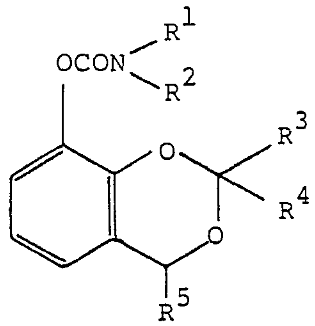

(I)

worin

$R^1$ Wasserstoff oder Methyl,

$R^2$, $R^3$ und $R^4$ unabhängig voneinander $C_{1-4}$-Alkyl und

$R^5$ Wasserstoff oder Methyl bedeuten.

Die Verbindungen der Formel I sind Schädlingsbekämpfungsmittel und eignen sich insbesondere zur Bekämpfung von Insekten, Milben, Tausendfüsslern, Nematoden und Schnecken. Somit umfasst die Erfindung auch Schädlingsbekämpfungsmittel, die als Wirkstoff eine oder mehrere der Verbindungen der Formel I enthalten, Verfahren zur Herstellung dieser Verbindungen sowie die Verwendung der Verbindungen bzw. Mittel zur Schädlingsbekämpfung.

The Merck Index, 9. Ausgabe (1976), Seite 1036 sowie K. H. Büchel, « Pflanzenschutz und Schädlingsbekämpfung », Georg Thieme Verlag, Stuttgart 1977, Seite 65 beschreiben den pestiziden Wirkstoff « Bendiocarb », das 2,2-Dimethyl-1,3-benzodioxol-4-ol-methyl-carbamat, die Wirksubstanz mehrerer Handelspräparate. Die erfindungsgemässen Verbindungen unterscheiden sich im wesentlichen von der bekannten Verbindung durch die Anwesenheit eines 1,3-Benzodioxan-Ringes anstelle eines 1,3-Benzodioxolan-Ringes.

Die französische Patentpublikation Nr. 2,359,136 beschreibt insektizid wirksame 3,4-Dihydro-2H-1,5-benzodioxepin-6-yl-carbamate, von denen sich die erfindungsgemässen Verbindungen im wesentlichen durch die Anwesenheit eines 1,3-Benzodioxan-Ringes anstelle eines 3,4-Dihydro-2H-1,5-benzodioxepin-Ringes unterscheiden.

In der obigen Definition der Reste in den Verbindungen der Formel I umfasst der Ausdruck « $C_{1-4}$-Alkyl » sowohl geradkettige als auch verzweigte Kohlenwasserstoffgruppen. Beispiele sind Methyl, Aethyl, n-Propyl, Isopropyl, n-Butyl, Isobutyl, sek. Butyl und tert. Butyl.

Da im Molekül asymmetrische Kohlenstoffatome vorhanden sein können, liegen in solchen Fällen optische Antipoden vor. Für den Fall, dass $R^3$ und $R^4$ verschiedene Bedeutungen besitzen und gleichzeitig $R^5$ Methyl bedeutet, können Stereoisomere vorliegen.

Die Formel I soll demnach alle oben angesprochenen möglichen isomeren Formen umfassen.

Unabhängig voneinander bedeuten : $R^2$ vorzugsweise Methyl oder n-Propyl, insbesondere Methyl ; $R^3$ vorzugsweise Methyl oder Aethyl, insbesondere Methyl ; $R^4$ vorzugsweise Methyl ; und $R^5$ vorzugsweise Wasserstoff.

Bevorzugt sind demnach diejenigen Verbindungen der Formel I, in der $R^1$ Wasserstoff oder Methyl, $R^2$ Methyl, $R^3$ und $R^4$ Methyl und $R^5$ Wasserstoff bedeuten.

Weiterhin sind Verbindungen der Formel I bevorzugt, in der $R^1$ Wasserstoff und $R^2$ Methyl oder n-Propyl bedeuten.

Eine besonders bevorzugte Verbindung der Formel I ist das 2,2-Dimethyl-1,3-benzodioxan-8-yl-N-methylcarbamat.

Das erfindungsgemässe Verfahren zur Herstellung der Verbindungen der Formel I ist dadurch gekennzeichnet, dass man

a) ein Phenol der allgemeinen Formel

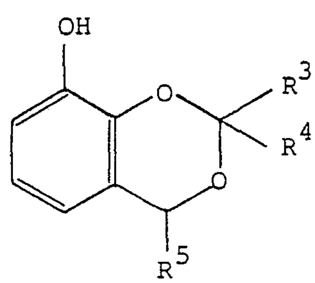

(II)

worin $R^3$, $R^4$ und $R^5$ die oben angegebenen Bedeutungen besitzen, mit einem Isocyanat der allgemeinen Formel

$$R^2N{=}C{=}O \quad (III)$$

worin $R^2$ die oben angegebene Bedeutung besitzt, umsetzt,

b) ein Phenol der allgemeinen Formel II, wie oben definiert, oder ein Alkalisalz davon, mit einem Carbamoylhalogenid der allgemeinen Formel

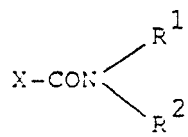

(IV)

worin $R^1$ und $R^2$ die oben angegebenen Bedeutungen besitzen und X ein Halogenatom, vorzugsweise Fluor oder Chlor, bedeutet, umsetzt, oder

c) ein Phenol der allgemeinen Formel II, wie oben definiert, mit Phosgen und anschliessend einem Amin der allgemeinen Formel

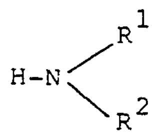

(V)

worin $R^1$ und $R^2$ die oben angegebenen Bedeutungen besitzen, umsetzt.

Die Umsetzung gemäss Verfahrensvariante a) führt zu Verbindungen der Formel I, worin $R^1$ Wasserstoff bedeutet, und erfolgt zweckmässigerweise in Gegenwart eines inerten Verdünnungsmittels, insbesondere eines organischen Lösungsmittels, sowie gegebenenfalls in Gegenwart eines Katalysators.

Zu den bevorzugten inerten organischen Lösungsmitteln gehören aliphatische und cyclische Aether, wie 1,2-Dimethoxyäthan, Tetrahydrofuran und Dioxan ; Ketone wie Diäthylketon, insbesondere Aceton und Methyläthylketon ; Nitrile wie Propionitril, insbesondere Acetonitril ; Formamide wie Dimethylformamid ; halogenierte Kohlenwasserstoffe, wie Methylenchlorid, Tetrachlorkohlenstoff und Chloroform ; Kohlenwasserstoffe, wie Petroläther, Toluol und n-Hexan ; und Ester, wie Essigsäureäthylester (Essigester).

Bevorzugte Katalysatoren für diese Verfahrensvariante sind tertiäre Basen, wie Triäthylamin, Pyridin und 1,4-Diazabicyclo(2,2,2)octan ; und zinnorganische Verbindungen, wie Dibutylzinndiacetat.

Die Reaktionstemperaturen können bei der Durchführung dieser Verfahrensvariante in einem grossen Bereich variiert werden. Im allgemeinen wird jedoch zwischen 0 °C und 100 °C gearbeitet, vorzugsweise zwischen 20 °C und 50 °C.

Auch die Umsetzung gemäss Verfahrensvariante b) erfolgt zweckmässigerweise in Gegenwart eines inerten Verdünnungsmittels, vorzugsweise eines organischen Lösungsmittels. Hierzu gehören vorzugsweise die im Zusammenhang mit der Verfahrensvariante a) aufgezählten Lösungsmittel.

Zudem wird die Umsetzung nach Verfahrensvariante b) zweckmässigerweise in Gegenwart eines Säurebinders vorgenommen. Man kann alle üblicherweise verwendbaren anorganischen und organischen Säurebinder zugeben. Hierzu gehören vorzugsweise Natriumhydrid ; Alkalicarbonate, wie Natriumcarbonat, Kaliumcarbonat und Natriumhydrogencarbonat ; niedere tertiäre Alkylamine, Cycloalkylamine und Arylalkylamine, wie Triäthylamin und Dimethylbenzylcyclohexylamin ; Pyridin ; und 1,4-Diazabicyclo(2,2,2)octan.

Die Reaktionstemperaturen können bei der Durchführung dieser Verfahrensvariante in einem grossen Bereich variiert werden. Im allgemeinen arbeitet man jedoch zwischen 0 °C und 100 °C, vorzugsweise zwischen 0 °C und 40 °C.

Man setzt vorzugsweise auf 1 Mol des Phenols der Formel II 1 bis 2 Mol an Carbamoylhalogenid der Formel IV ein. Zudem hat es sich als vorteilhaft erwiesen, den Säurebinder in geringem Ueberschuss (bis ca. 30 Gewichtsprozent) einzusetzen. Die Isolierung der so erhaltenen Verbindung der Formel I kann in üblicher Weise erfolgen.

Wie im Falle der Verfahrensvariante b) erfolgt die Umsetzung gemäss Verfahrensvariante c) zweckmässigerweise in Gegenwart eines inerten Verdünnungsmittels, insbesondere eines organischen Lösungsmittels, sowie in Gegenwart eines Säurebinders.

Als bevorzugte organische Lösungsmittel kommen insbesondere aliphatische und cyclische Aether, wie 1,2-Dimethoxyäthan, Tetrahydrofuran und Dioxan ; Nitrile, wie Acetonitril ; Formamide, wie Dimethylformamid ; und Kohlenwasserstoffe, wie Toluol, in Frage. Man kann alle üblicherweise verwendbaren anorganischen und organischen Säurebinder zugeben, zu denen vorzugsweise die im Zusammenhang mit der Verfahrensvariante b) oben erwähnten gehören.

Die Reaktionstemperaturen können bei der Durchführung dieser Verfahrensvariante in einem grossen Bereich variiert werden. Im allgemeinen liegen sie zweckmässigerweise zwischen 0 °C und 100 °C, vorzugsweise zwischen 0 °C und 40 °C.

Zweckmässigerweise wird das Verfahren als Eintopfverfahren durchgeführt. Man setzt erst Phosgen mit dem Phenol der Formel II um und gibt dann zum Reaktionsgemisch das Amin der Formel V zu. Es hat sich als vorteilhaft erwiesen, auf 1 Mol des Phenols der Formel II 2 bis 2,5 Mol Phosgen und 1 bis 1,5 Mol des Amins der Formel V einzusetzen. Ferner wird der Säurebinder vorzugsweise in geringem Ueberschuss, z. B. bis ca. 30 Gewichtsprozent, verwendet. Die Isolierung der so erhaltenen Verbindung der Formel I kann in üblicher Weise erfolgen.

Sofern keine gezielte Synthese zur Isolierung reiner Isomerer durchgeführt wird, fällt das Produkt normalerweise als Gemisch zweier oder mehrerer Isomerer an. Die Isomeren können nach an sich bekannten Methoden aufgetrennt werden.

Die als Ausgangsmaterialien verwendeten Phenole der Formel II sind neue Verbindungen und bilden einen weiteren Gegenstand der vorliegenden Erfindung. Bezüglich der bevorzugten Bedeutungen von $R^3$-$R^5$ gilt das oben Gesagte. Eine besonders bevorzugte Verbindung der Formel II ist das 2,2-Dimethyl-1,3-benzodioxan-8-ol.

Die Verbindungen der Formel II können beispielsweise hergestellt werden, indem man einen Methyläther der allgemeinen Formel

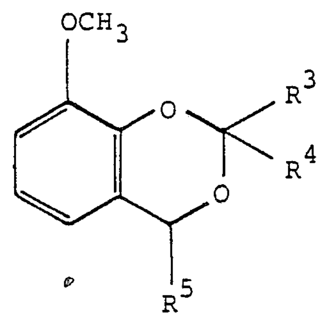

(VI)

worin $R^3$, $R^4$ und $R^5$ die oben angegebenen Bedeutungen besitzen, zweckmässigerweise in Anwesenheit eines Verdünnungsmittels einer Aetherspaltung unterwirft.

Die dabei verwendeten Aetherspaltungsreagenzien müssen eine hohe Selektivität für die Methoxygruppe aufweisen. Als besonders vorteilhaft haben sich Alkalisalze von Mercaptanen resp. Thiophenolen erwiesen, wie beispielsweise von Aethylmercaptan, Thiophenol und p-Thiokresol.

Als Verdünnungsmittel eignen sich insbesondere dipolare aprotische Lösungsmittel, wie Dimethylformamid und Dimethylsulfoxyd, welche gegebenenfalls auch zusammen mit weiteren inerten organischen Lösungsmitteln eingesetzt werden können.

Die Reaktionstemperaturen liegen zweckmässigerweise zwischen 100 °C und 200 °C, vorzugsweise bei ca. 150 °C.

Zur Isolierung der so erhaltenen Verbindung der Formel II kann das Lösungsmittel entfernt werden, z. B. durch Abdestillieren, und der Rückstand gegebenenfalls nach Ansäuern in ein geeignetes Lösungsmittel aufgenommen, mit Wasser gewaschen, die Lösung über einem geeigneten Trocknungsmittel, z. B. wasserfreiem Natriumsulfat, getrocknet und nach Abdestillieren des Lösungsmittels das Produkt umkristallisiert oder chromatographisch gereinigt werden.

Die Methyläther der Formel VI sind ebenfalls neu und bilden ebenfalls einen Gegenstand der vorliegenden Erfindung. Auch in diesem Fall gilt bezüglich der bevorzugten Bedeutungen von $R^3$-$R^5$ das oben Gesagte. Eine besonders bevorzugte Verbindung der Formel VI ist das 2,2-Dimethyl-8-methoxy-1,3-benzodioxan.

Die Verbindungen der Formel VI können beispielsweise hergestellt werden, indem man eine Dihydroxyverbindung der allgemeinen Formel

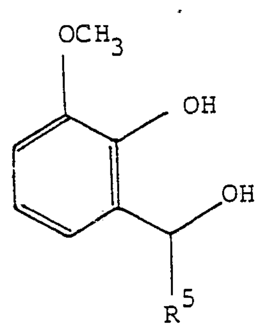

(VII)

worin $R^5$ die oben angegebene Bedeutung besitzt, mit einer Carbonylverbindung der allgemeinen Formel

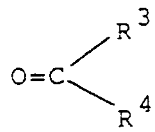

(VIII)

worin $R^3$ und $R^4$ die oben angegebenen Bedeutungen besitzen, umsetzt.

Diese Reaktion wird in an sich bekannter Weise, zweckmässigerweise in Gegenwart eines Verdünnungsmittels, insbesondere eines organischen Lösungsmittels, in Gegenwart eines Katalysators, wie p-Toluolsulfonsäure, und gegebenenfalls unter Verwendung eines Wasserabscheiders oder eines wasserbindenden Mittels durchgeführt.

Als organische Lösungsmittel können beispielsweise aromatische Kohlenwasserstoffe, wie Benzol, Toluol und Xylol ; halogenierte Kohlenwasserstoffe, wie Chlorbenzol, Chloroform und 1,2-Dichloräthan, und aliphatische oder cyclischer Aether, wie 1,2-Dimethoxyäthan und Dioxan, verwendet werden.

Die Reaktionstemperaturen können in einem breiten Bereich variiert werden. Im allgemeinen wird jedoch zwischen 20 °C und der Siedetemperatur des verwendeten Lösungsmittels gearbeitet.

Für die Herstellung derjenigen Verbindungen der Formel VI, worin $R^3$ und $R^4$ Methyl bedeuten, hat sich die Verwendung von 2,2-Dimethoxypropan an Stelle von Aceton als Verbindung der Formel VIII ebenfalls als zweckmässig erwiesen.

Die Hydroxyverbindungen der Formel VII sind entweder bekannt oder können nach an sich bekannten Methoden hergestellt werden.

Die als Ausgangsmaterialien verwendeten Isocyanate der Formel III sind entweder bekannt oder können nach an sich bekannten Methoden, z. B. durch Umsetzung des diesbezüglichen Amins $R^2NH_2$ mit Phosgen und anschliessendes Erhitzen, hergestellt werden. Beispiele solcher bekannter Isocyanate sind Methylisocyanat, Aethylisocyanat, Isopropylisocyanat, tert.-Butylisocyanat und Allylisocyanat.

Die als Ausgangsmaterialien verwendeten Carbamoylhalogenide der Formel IV sind ebenfalls entweder bekannt oder können nach an sich bekannten Methoden, z. B. durch Umsetzung des diesbezüglichen Amins der Formel V mit Phosgen, hergestellt werden. Beispiele solcher bekannter Carbamoylhalogenide sind Methylcarbamoylchlorid, Methyläthylcarbamoylchlorid, Dimethylcarbamoylchlorid und Methylcarbamoylfluorid.

Die als Ausgangsmaterialien verwendeten Amine der Formel V sind ebenfalls entweder bekannt oder können nach an sich bekannten Methoden hergestellt werden. Beispiele solcher bekannter Amine sind Methylamin, Aethylamin, Dimethylamin, Methyläthylamin, N-Allylmethylamin und N-Methylpropargylamin.

Die Verbindungen der Formel I sind ganz allgemein als Pestizide von Wert. Sie erweisen sich als besonders wertvoll zur Bekämpfung von Insekten, Milben, Tausendfüsslern, Nematoden und Schnecken, insbesondere von

Coleoptera (Käfern)

wie z. B. Epilachna spp., Leptinotarsa decemlineata, Anthonomus spp., Conotrachelus nenuphar, Lema spp., Lissorhoptrus oryzaephilus, Phyllotreta spp., Psylliodes chrysocephala, Meligethes aenus, Phyllotreta spp., Popilla japonica, Hypera postica, Ataenius spp., Oulema oryzae, Chaetocnema spp., Ceutorrhynchus assimilis, Agriotes spp., Otiorhynchus sulcatus, Melolontha melolontha, Diabrotica und anderen Boden-Coleopteren ;

Lepidoptera (Schmetterlingen)

wie z. B. Laspeyresia spp., Adoxophyes orana, Tortrix viridana, Cheimatobia brumata, Lyonetia clerkella, Operophtera brumata, Lithocolletis blancardella, Porthetria dispar, Mamestra brassicae, Agrotis spp., Plutella spp., Pieris brassicae, Choristoneura fumiferana, Heliothis spp., Spodoptera spp., Pectinophora gossipiella, Chilo spp., Ostrinia nubilalis, Clysia ambiguella, Lobesia botrana, Ephestia kuehniella, Tryporyza spp., Diatraea spp. ;

Diptera (Zweiflüglern)

wie z. B. Drosophila melanogaster, Ceratitis spp., Oscinella frit, Dacus spp. und Rhagoletis spp., Leatherjacket spp., Sciara spp., Phorbia spp. und Megasetia agarici, Delia antiqua, Pegomyia hyoscyami, Musca domestica, Aedes spp., Culex spp., Anopheles spp. ;

Homoptera (Blattläusen)

wie z. B. Aphis fabae, Myzus persicae und weiteren Arten dieser Gattungen, Acyrtosiphon spp., Rhophalosiphon spp., Schizaphis spp., Dysaphis spp., Eriosoma spp., Macrosiphum spp., Adelges spp., Sitobion avenae, Metopolophium spp., Brachycandus spp., Hyalopterus spp., Brevicorynae spp., Phorodon spp. sowie Schild- und Schmierläusen wie z. B. Aonidiella aurantii, Pseudococcus spp., Rhizoecus spp. sowie Zikaden wie z. B. Nephotettix spp., Laodelphax spp., Nilaparvata spp., Sogatella spp. und Erythroneura spp., Aleurodidae wie z. B. Trialeurodes vaporariorum, Bemisia spp. ;

Heteroptera (Wanzen)

wie z. B. Dysdercus cingulatus, Lygus spp. ;

Thysanoptera (Fransenflüglern)
wie verschiedenen Thrips-Arten ;
Collembola (Springschwänzen)
wie z. B. Onychiurus spp. ;
Orthoptera (Geradflüglern)
wie z. B. Blattella germanica, Blatta orientalis ;
Acarina (Milben)
z. B. Tetranychidae (Spinnmilben) wie z. B. Tetranychus urticae, Tetranychus cinnabarinus, Panonychus ulmi und anderen Tetranychiden, Eriophyiden wie Phyllocoptruta oleivora, ferner Zwecken wie z. B. Amblyomma spp., Boophilus spp. und anderen Ixodoiden ;
Nematoda (Fadenwürmern)
wie z. B. Ditylenchus dipsaci, Meloidogyne incognita, Pratylenchus penetrans, Aphelenchoides rizemabosi und Globodera rostochiensis ;
Myriapoda (Tausendfüsslern)
wie z. B. Scutigerella spp.

Die Verbindungen der Formel I wirken als Kontakt- und Frassgifte und zeichnen sich durch gute systemische Wirkung aus. Darüber hinaus weisen sie eine gute Persistenz der biologischen Wirkung auf Blattwerk und im Erdboden, sowie gute Regenfestigkeit auf. Die Verbindungen sind wirksam gegen verschiedene Schädlinge mit Resistenz gegen Phosphorsäureester-Pestizide.

Das erfindungsgemässe Schädlingsbekämpfungsmittel ist dadurch gekennzeichnet, dass es eine wirksame Menge mindestens einer Verbindung der allgemeinen Formel I, wie oben definiert, sowie Formulierungshilfsstoffe enthält. Das Mittel enthält zweckmässigerweise zumindest einen der folgenden Formulierungshilfsstoffe :

Feste Trägerstoffe ; Lösungs- bzw. Dispersionsmittel ; Tenside (Netz- und Emulgiermittel) ; Dispergatoren (ohne Tensidwirkung) ; und Stabilisatoren.

Unter Verwendung solcher und anderer Hilfsstoffe können die Verbindungen der Formel I, also die pestiziden Wirkstoffe, in die üblichen Formulierungen übergeführt werden, wie Lösungen, Suspensionen, Emulsionen, emulgierbare Konzentrate, Pasten, Schäume, Stäube, Pulver und Granulate.

Als feste Trägerstoffe kommen im wesentlichen in Frage : natürliche Mineralstoffe, wie Kaolin, Tonerden, Kieselgur, Talkum, Bentonit, Kreide, Kalkstein, Quarz, Dolomit, Attapulgit, Montmorillonit und Diatomeenerde ; synthetische Mineralstoffe, wie hochdisperse Kieselsäure, Aluminiumoxid und Silikate ; organische Stoffe, wie Cellulose, Stärke, Harnstoff und Kunstharze ; und Düngemittel, wie Phosphate und Nitrate, wobei solche Trägerstoffe z. B. als Stäube, Pulver oder Granulate vorliegen können.

Als Lösungs- bzw. Dispersionsmittel kommen im wesentlichen in Frage : Aromaten, wie Toluol, Xylole, Benzol und Alkylnaphthaline ; chlorierte Aromaten und chlorierte aliphatische Kohlenwasserstoffe, wie Chlorbenzole, Chloräthylene und Methylenchlorid ; aliphatische Kohlenwasserstoffe, wie Cyclohexan und Paraffine, z. B. Erdölfraktionen ; Alkohole, wie Butanol und Glykol, sowie deren Aether und Ester ; Ketone, wie Aceton, Methyläthylketon, Methylisobutylketon und Cyclohexanon ; und stark polare Lösungsmittel, wie Dimethylformamid, N-Methylpyrrolidon und Dimethylsulfoxid, wobei solche Lösungs- bzw. Dispersionsmittel vorzugsweise Flammpunkte von mindestens 30 °C und Siedepunkte von mindestens 50 °C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z. B. Dichlordifluormethan. Im Falle der Benutzung von Wasser als Lösungsmittel können z. B. auch organische Lösungsmittel als Hilfslösungsmittel verwendet werden.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid ; Fettsäureester und -äther von Zuckern oder mehrwertigen Alkoholen ; die Produkte, die aus Zuckern oder mehrwertigen Alkoholen durch Kondensation mit Aethylenoxid erhalten werden ; Blockpolymere von Aethylenoxid und Propylenoxid ; oder Alkyldimethylaminoxide.

Die Tenside können auch anionische Verbindungen darstellen, wie Seifen ; Fettsulfatester, z. B. Dodecylnatriumsulfat, Octadecylnatriumsulfat und Cetylnatriumsulfat ; Alkylsulfonate, Arylsulfonate und fettaromatische Sulfonate, wie Alkylbenzolsulfonate, z. B. Calcium-Dodecylbenzolsulfonat, und Butylnaphthalinsulfonate ; und komplexere Fettsulfonate, z. B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage : Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z. B.

Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z. B. Epichlorhydrin, Phenylglycidäther und Soyaepoxide ; Antioxidantien, z. B. Gallussäureester und Butylhydroxytoluol ; UV-Absorber, z. B. substituierte Benzophenone, Diphenylacrylnitrilsäureester und Zimtsäureester ; und Deaktivatoren, z. B. Salze der Aethylendiaminotetraessigsäure und Polyglykole.

Die erfindungsgemässen Schädlingsbekämpfungsmittel können neben den Wirkstoffen der Formel I auch andere Wirkstoffe enthalten, z. B. anderweitige Schädlingsbekämpfungsmittel, Schädlingslockstoffe, Fungizide, Bakterizide, Herbizide, Pflanzenwachstumsregulatoren und Düngemittel. Solche Kombinationsmittel eignen sich zur Verstärkung der Aktivität bzw. zur Verbreiterung des Wirkungsspektrums. Gegebenenfalls können dadurch auch Unzulänglichkeiten von bisher bekannten zugesetzten Mitteln ausgeglichen werden.

Die erfindungsgemässen Schädlingsbekämpfungsmittel enthalten im allgemeinen zwischen 0,000 5 und 95 Gewichtsprozent der Verbindung bzw. Verbindungen der Formel I als Wirkstoff, vorzugsweise zwischen 0,5 und 90 Gewichtsprozent. Sie können in einer Form vorliegen, die sich für die Lagerung und den Transport eignet. In solchen Formen, z. B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zur Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im allgemeinen 5 bis 95 Gewichtsprozent, vorzugsweise 10 bis 90 Gewichtsprozent, der Verbindung bzw. Verbindungen der Formel I. Als Anwendungsformen kommen u. a. gebrauchsfertige Lösungen, Emulsionen, Suspensionen, Schäume, Pulver, Pasten, Stäubemittel und Granulate in Frage. Die Wirkstoffkonzentrationen in solchen anwendungsfertigen Zubereitungen können in grossen Bereichen variiert werden. In Spritzbrühen können z. B. Konzentrationen zwischen 0,000 5 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen. Granulate enthalten vorzugsweise von 1 bis 20 Gewichtsprozent der Verbindung bzw. Verbindungen der Formel I als Wirkstoff.

Die erfindungsgemässen Schädlingsbekämpfungsmittel können dadurch hergestellt werden, dass man mindestens eine Verbindung der allgemeinen Formel I mit Formulierungshilfsstoffen vermischt.

Die Herstellung der Mittel kann in bekannter Weise durchgeführt werden, z. B. durch Vermischen des Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z. B. durch Zusammenmahlen ; oder man kann den festen Trägerstoff mit einer Lösung oder Suspension des Wirkstoffs imprägnieren und dann das Lösungs- oder Suspensionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z. B. als Spritzmittel eignen, übergeführt werden können.

Die Verbindungen der Formel I können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann der Verbindung der Formel I in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise enthält man emulgierbare Konzentrate bzw. gebrauchsfertige Emulsionen.

Das erfindungsgemässe Verfahren zur Bekämpfung von Schädlingen ist dadurch gekennzeichnet, dass man die zu schützenden Gegenstände oder die Schädlinge selbst mit einer erfindungsgemässen Verbindung bzw. einem erfindungsgemässen Schädlingsbekämpfungsmittel behandelt. Dieses Anwendungsverfahren kann durch Boden- oder Blattapplikation, bzw. durch Applikation auf die zu schützenden Tiere, Vorräte oder Materialien, je nach Art der zu bekämpfenden Schädlinge, durchgeführt werden. Die Bekämpfung wird beispielsweise durch Kontakt oder durch Einnahme mit der Nahrung erzielt.

Die Anwendung kann in konventioneller Weise geschehen, z. B. durch Verspritzen, Versprühen, Vernebeln, Verstäuben, Verstreuen, Eindrillen, Verräuchern, Giessen, Beizen oder Inkrustieren.

Pulverförmige Präparate können z. B. als Stäubemittel mit Hilfe der üblichen Verstäubegeräte auf die Schädlinge bzw. auf die zu schützenden Gegenstände, z. B. Pflanzen oder Tiere, aufgebracht werden. Wässrige Suspensionen sind z. B. als Spritzmittel anwendbar.

Die nachfolgenden Beispiele dienen zur näheren Erläuterung der Erfindung.

I. Herstellung der Wirkstoffe der Formel I

Beispiel 1

10,81 g 2,2-Dimethyl-1,3-benzodioxan-8-ol werden in 70 ml n-Hexan-Essigester (6 : 1) gelöst und mit 0,5 ml Dibutylzinn-diacetat versetzt. Es wird innerhalb von 30 Minuten eine Lösung von 3,77 g Methylisocyanat in 80 ml n-Hexan zugetropft. Das Produkt fällt aus, und die Temperatur steigt auf ca. 30 °C an. Man rührt noch 1 Stunde weiter. Die Kristalle werden abgenutscht, mit n-Hexan gut gewaschen und am Hochvakuum getrocknet. Man erhält reines 2,2-Dimethyl-1,3-benzodioxan-8-yl-N-methylcarbamat ; Smp. 147-148 °C.

In Analogie zu obigem Verfahren erhält man aus :

2-Aethyl-2-methyl-1,3-benzodioxan-8-ol und Methylisocyanat das 2-Aethyl-2-methyl-1,3-benzodioxan-8-yl-N-methylcarbamat ; Smp. 78-80 °C.

2,2,4-Trimethyl-1,3-benzodioxan-8-ol und Methylisocyanat das 2,2,4-Trimethyl-1,3-benzodioxan-8-yl-N-methylcarbamat ; Smp. 152-154 °C.

2,2-Dimethyl-1,3-benzodioxan-8-ol und Aethylisocyanat das 2,2-Dimethyl-1,3-benzodioxan-8-yl-N-äthylcarbamat ; Smp. 98-100 °C.

2,2-Dimethyl-1,3-benzodioxan-8-ol und n-Propylisocyanat das 2,2-Dimethyl-1,3-benzodioxan-8-yl-N-(n-propyl) carbamat ; Smp. 99-101 °C.

2,2-Diäthyl-1,3-benzodioxan-8-ol und Methylisocyanat das 2,2-Diäthyl-1,3-benzodioxan-8-yl-N-methylcarbamat ; Smp. 86-89 °C.

Beispiel 2

0,53 g Natriumhydrid-Dispersion (55-60 %ig in Oel) werden in 10 ml N,N-Dimethylformamid vorgelegt. Zum Gemisch wird innerhalb von 15 Minuten bei Raumtemperatur eine Lösung von 2,0 g 2,2-Dimethyl-1,3-benzodioxan-8-ol in 15 ml N,N-Dimethylformamid zugetropft, wobei die Temperatur auf 30 °C ansteigt. Anschliessend wird 4 Stunden bei Raumtemperatur gerührt. Nun werden 1,19 g N,N-Dimethylcarbaminsäurechlorid innerhalb von 10 Minuten zugetropft, und das ganze Gemisch wird 1 Stunde bei 40 °C und während 16 Stunden bei Raumtemperatur gerührt. Zur Aufarbeitung wird auf Eiswasser gegossen und mit Essigester extrahiert. Die Extrakte werden mit Wasser und Kochsalzlösung gewaschen, über wasserfreiem Natriumsulfat getrocknet und eingedampft. Nach Chromatographie an Kieselgel mit n-Hexan-Essigester (2 : 1) erhält man reines 2,2-Dimethyl-1,3-benzodioxan-8-yl-N,N-dimethylcarbamat ; $n_D^{20}$ : 1,517 0.

II. Herstellung der Ausgangsmaterialien

Beispiel 3

35,56 g Natriumhydrid-Dispersion (55-60 %ig in Oel) werden in 500 ml N,N-Dimethylformamid vorgelegt und 50,64 g Aethylmercaptan, gelöst in 100 ml N,N-Dimethylformamid, innerhalb von 60 Minuten zugetropft. Es wird ein Temperaturanstieg auf 40 °C beobachtet. Anschliessend wird 30 Minuten bei Raumtemperatur gerührt, wobei eine gelbe, klare Lösung erhalten wird. Nun wird eine Lösung von 63,22 g 2,2-Dimethyl-8-methoxy-1,3-benzodioxan in 215 ml N,N-Dimethylformamid zugegeben und das Ganze auf Rückflusstemperatur erhitzt. Nach einstündiger Reaktionszeit lässt man das Gemisch abkühlen und destilliert die Hauptmenge des Lösungsmittels unter vermindertem Druck ab. Der Rückstand wird vorsichtig auf 1 l Eiswasser gegossen und tropfenweise mit 48,94 g Essigsäure versetzt. Anschliessend wird das Gemisch mit Essigester extrahiert, und die Extrakte werden mit Wasser und Kochsalzlösung gewaschen, über wasserfreiem Natriumsulfat getrocknet und eingedampft. Durch Chromatographie an Kieselgel mit n-Hexan/Essigester (4 : 1) als Elutionsmittel erhält man reines 2,2-Dimethyl-1,3-benzodioxan-8-ol ; Smp. 32-34 °C.

In Analogie zu obigem Verfahren erhält man aus :

2-Aethyl-2-methyl-8-methoxy-1,3-benzodioxan das 2-Aethyl-2-methyl-1,3-benzodioxan-8-ol ; Smp. 22-25 °C.

2,2,4-Trimethyl-8-methoxy-1,3-benzodioxan das 2,2,4-Trimethyl-1,3-benzodioxan-8-ol ; Smp. 86-90 °C.

2,2-Diäthyl-8-methoxy-1,3-benzodioxan das 2,2-Diäthyl-1,3-benzodioxan-8-ol ; $n_D^{20}$ : 1,519 3.

Beispiel 4

80,16 g 2-Hydroxy-3-methoxy-benzylalkohol und 108,32 g 2,2-Dimethoxypropan werden in Gegenwart von 2 g Toluol-4-sulfonsäure-monohydrat in 860 ml Benzol unter Rühren zum Sieden erhitzt. Nach 30 Minuten (Rückflusstemperatur) ist die Reaktion beendet. Unter Rühren werden 5 g Kaliumcarbonat zugesetzt, und nach 15 Minuten wird das Reaktionsgemisch auf 1 l Eiswasser gegossen. Die benzolische Lösung wird der Reihe nach mit Wasser, halb gesättigter- und gesättiger Kochsalzlösung gewaschen, über wasserfreiem Natriumsulfat getrocknet und am Rotavapor eingedampft. Durch Chromatographie an Kieselgel mit n-Hexan-Essigester (9 : 1) erhält man reines 2,2-Dimethyl-8-methoxy-1,3-benzodioxan ; $n_D^{20}$ : 1,535 0.

Beispiel 5

3,08 g 2-Hydroxy-3-methoxy-benzylalkohol und 50 ml Methyläthylketon werden in Gegenwart von 50 mg Toluol-4-sulfonsäure-monohydrat in 50 ml Benzol unter Rühren zum Sieden erhitzt. Nach 30 Minuten wird die Heizung entfernt, und unter Rühren werden 0,5 g Kaliumcarbonat zugesetzt. Nach Verdünnen mit ca. 100 ml Diäthyläther wird das Reaktionsgemisch auf Eiswasser gegossen. Die organische Lösung wird der Reihe nach mit halb gesättigter und gesättigter Kochsalzlösung gewaschen, über wasserfreiem Natriumsulfat getrocknet und am Rotavapor eingedampft. Durch Chromatographie an Kieselgel mit n-Hexan-Essigester (9 : 1) erhält man reines 2-Aethyl-2-methyl-8-methoxy-1,3-benzodioxan ; $n_D^{20}$ : 1,530 8.

In Analogie zu obigem Verfahren erhält man aus :

2-Hydroxy-3-methoxy-benzylalkohol und Aceton in Benzol als Lösungsmittel das 2,2-Dimethyl-8-methoxy-1,3-benzodioxan ; $n_D^{20}$ : 1,535 0.

2-Hydroxy-3-methoxy-α-methyl-benzylalkohol und Aceton das 2,2,4-Trimethyl-8-methoxy-1,3-benzodioxan, das als Rohprodukt weiterverarbeitet wird.

2-Hydroxy-3-methoxy-benzylalkohol und Diäthylketon das 2,2-Diäthyl-8-methoxy-1,3-benzodioxan, das als Rohprodukt weiterverarbeitet wird.

Beispiel 6

5,1 g Magnesiumspäne werden in 20 ml absolutem Diäthyläther vorgelegt und 1/10 einer Lösung von 32,79 g Methyljodid in 100 ml absolutem Diäthyläther auf einmal zugegeben. Die Reaktion springt sofort an. Nun wird der Rest der Methyljodid-Lösung so zugetropft, dass das Reaktionsgemisch unter stetem Rückfluss bleibt. Nach erfolgter Zugabe wird 2 Stunden nachgerührt. Nun werden 10,65 g 2-Hydroxy-3-methoxy-benzaldehyd, gelöst in 80 ml absolutem Diäthyläther, so zugetropft, dass das Gemisch unter stetem Rückfluss bleibt. Nach erfolgter Zugabe wird noch 30 Minuten nachgerührt, hierauf wird unter Rühren eine gesättigte Ammoniumchloridlösung langsam zugetropft (exotherme Reaktion). Es bildet sich ein klebriger Niederschlag, der mit Essigester aufgeschlämmt und auf Eiswasser gegossen wird. Unter Rühren wird durch Zugabe von konzentrierter Salzsäure sauer gestellt, wobei klare Phasentrennung eintritt. Die organische Phase wird abgetrennt, mit Wasser und Kochsalzlösung gewaschen, über wasserfreiem Natriumsulfat getrocknet und eingedampft. Durch Chromatographie an Kieselgel mit n-Hexan-Essigester (2 : 1) erhält man reinen 2-Hydroxy-3-methoxy-α-methyl-benzylalkohol ; $n_D^{20}$ : 1,547 8.

III. Formulierungsbeispiele

Beispiel 7

| Spritzpulver | Gew.% |
|---|---|
| Wirkstoff der Formel I | 50 |
| Kieselsäure, hydratisiert (ca. 87 % $SiO_2$) | 5 |
| Natrium-laurylsulfat | 1 |
| Natrium-lignosulfonat | 2 |
| Kaolin, hauptsächlich $Al_2[Si_2O_5]$ $(OH)_4$ | 42 |
| | 100 |

Der Wirkstoff wird mit den übrigen Formulierungskomponenten in einer geeigneten Vorrichtung homogen vermischt. Das entstandene Pulver wird nun in einem geeigneten Mahlaggregat (z. B. Stiften-, Hammer-, Kugel- oder Luftstrahlmühle) auf eine für eine optimale biologische Wirksamkeit notwendige Teilchengrösse feingemahlen und hernach nochmals gemischt. Das nun vorliegende Spritzpulver wird durch Wasser spontan benetzt und ergibt gut schwebefähige, gebrauchsfertige Spritzbrühen.

Beispiel 8

| Granulat | Gew.% |
| --- | --- |
| Wirkstoff der Formel I | 5 |
| Tetranatriumsalz der Aethylendiamino-tetraessigsäure ($Na_4$-AeDTA) | 1 |
| Bimssteingranulat 0,6-1,0 mm | 94 |
| | 100 |

In einem geeigneten Mischwerk wird das Bimssteingranulat vorgelegt und unter stetigem Rühren eine wässrige Lösung des $Na_4$-AeDTA aufgesprüht. Das Gemisch wird bei 110 °C getrocknet und hernach der Wirkstoff, gelöst in einem geeigneten Lösungsmittel (wie z. B. Methylenchlorid), auf das trockene Gemisch aufgesprüht. Das Lösungsmittel wird durch Erwärmung verdampft. Es entsteht ein gut schüttbares Granulat, welches von Hand, mit geeigneten Granulatstreuern oder sogar vom Flugzeug aus, auf den Boden oder ins Wasser ausgebracht werden kann. Die poröse Struktur des Bimssteines bewirkt in vielen Fällen eine erwünschte verlangsamte Abgabe des Wirkstoffes über längere Zeit.

**Patentansprüche** (für die Vertragsstaaten : BE, CH, DE, FR, GB, IT, LI, LU, NL, SE)

1. Verbindungen der allgemeinen Formel

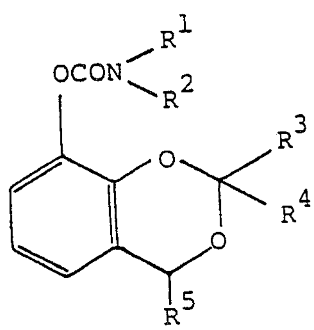

(I)

worin
$R^1$ Wasserstoff oder Methyl,
$R^2$, $R^3$ und $R^4$ unabhängig voneinander $C_{1-4}$-Alkyl und
$R^5$ Wasserstoff oder Methyl bedeuten.

2. Verbindungen nach Anspruch 1, worin $R^1$ Wasserstoff oder Methyl, $R^2$ Methyl, $R^3$ und $R^4$ je Methyl und $R^5$ Wasserstoff bedeuten.

3. Verbindungen nach Anspruch 1, worin $R^1$ Wasserstoff und $R^2$ Methyl oder n-Propyl bedeuten.

4. 2,2-Dimethyl-1,3-benzodioxan-8-yl-N-methylcarbamat.

5. Eine Verbindung nach Anspruch 1, ausgewählt aus :

2-Aethyl-2-methyl-1,3-benzodioxan-8-yl-N-methylcarbamat,
2,2,4-Trimethyl-1,3-benzodioxan-8-yl-N-methylcarbamat,
2,2-Dimethyl-1,3-benzodioxan-8-yl-N-äthylcarbamat,
2,2-Dimethyl-1,3-benzodioxan-8-yl-N-(n-propyl)carbamat und
2,2-Diäthyl-1,3-benzodioxan-8-yl-N-methylcarbamat.

6. 2,2-Dimethyl-1,3-benzodioxan-8-yl-N,N-dimethylcarbamat.

7. Eine Verbindung nach Anspruch 1 als Schädlingsbekämpfungsmittel.

8. Schädlingsbekämpfungsmittel, dadurch gekennzeichnet, dass es eine wirksame Menge mindestens einer Verbindung der allgemeinen Formel

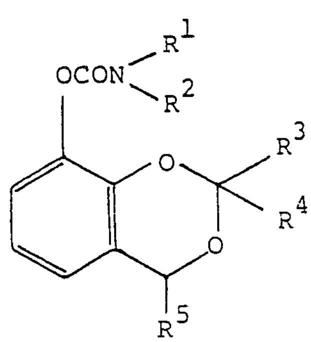

(I)

worin

$R^1$ Wasserstoff oder Methyl,

$R^2$, $R^3$ und $R^4$ unabhängig voneinander $C_{1-4}$-Alkyl und

$R^5$ Wasserstoff oder Methyl bedeuten,

sowie Formulierungshilfsstoffe enthält.

9. Schädlingsbekämpfungsmittel nach Anspruch 8, dadurch gekennzeichnet, dass es eine wirksame Menge 2,2-Dimethyl-1,3-benzodioxan-8-yl-N-methylcarbamat sowie Formulierungshilfsstoffe enthält.

10. Verfahren zur Herstellung von Verbindungen der allgemeinen Formel

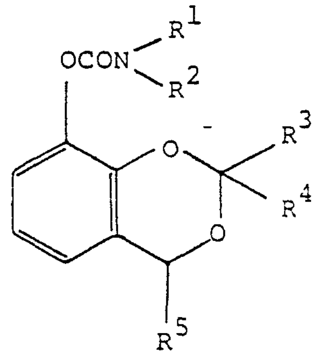

(I)

worin

$R^1$ Wasserstoff oder Methyl,

$R^2$, $R^3$ und $R^4$ unabhängig voneinander $C_{1-4}$-Alkyl und

$R^5$ Wasserstoff oder Methyl bedeuten,

dadurch gekennzeichnet, dass man

a) ein Phenol der allgemeinen Formel

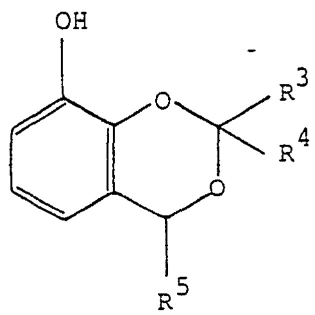

(II)

worin $R^3$, $R^4$ und $R^5$ die oben angegebenen Bedeutungen besitzen, mit einem Isocyanat der allgemeinen Formel

$$R^2N=C=O \qquad \text{(III)}$$

worin $R^2$ die oben angegebene Bedeutung besitzt, umsetzt,

b) ein Phenol der allgemeinen Formel II, wie oben definiert, oder ein Alkalisalz davon, mit einem Carbamoylhalogenid der allgemeinen Formel

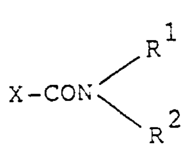

(IV)

worin $R^1$ und $R^2$ die oben angegebenen Bedeutungen besitzen und X ein Halogenatom bedeutet, umsetzt, oder

c) ein Phenol der allgemeinen Formel II, wie oben definiert, mit Phosgen und anschliessend einem Amin der allgemeinen Formel

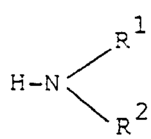

(V)

worin $R^1$ und $R^2$ die oben angegebenen Bedeutungen besitzen, umsetzt.

11. Verfahren zur Bekämpfung von Schädlingen, dadurch gekennzeichnet, dass man die zu schützenden Gegenstände oder die Schädlinge selbst mit mindestens einer der in den Ansprüchen 1 bis 6 genannten Verbindungen bzw. mit einem in Anspruch 8 oder 9 genannten Schädlingsbekämpfungsmittel behandelt.

12. Verwendung einer der in den Ansprüchen 1 bis 6 genannten Verbindungen bzw. eines in Anspruch 8 oder 9 genannten Schädlingsbekämpfungsmittels zur Bekämpfung von Schädlingen.

13. Verbindungen der allgemeinen Formel

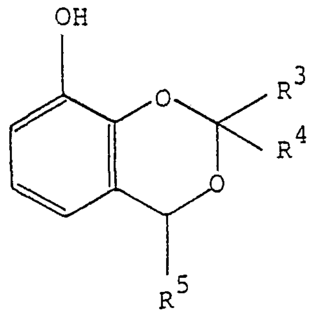

(II)

worin

$R^3$ und $R^4$ unabhängig voneinander $C_{1-4}$-Alkyl und

$R^5$ Wasserstoff oder Methyl bedeuten.

14. 2,2-Dimethyl-1,3-benzodioxan-8-ol.

15. Verbindungen der allgemeinen Formel

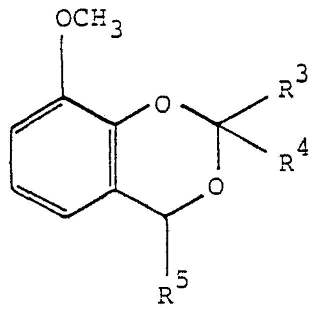

(VI)

worin

$R^3$ und $R^4$ unabhängig voneinander $C_{1-4}$-Alkyl und

$R^5$ Wasserstoff oder Methyl bedeuten.

16. 2,2-Dimethyl-8-methoxy-1,3-benzodioxan.

**Patentansprüche** (für den Vertragsstaat AT)

1. Schädlingsbekämpfungsmittel, dadurch gekennzeichnet, dass es eine wirksame Menge mindestens einer Verbindung der allgemeinen Formel

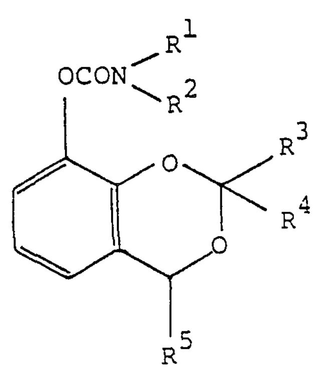

(I)

worin

$R^1$ Wasserstoff oder Methyl,

$R^2$, $R^3$ und $R^4$ unabhängig voneinander $C_{1-4}$-Alkyl und
$R^5$ Wasserstoff oder Methyl bedeuten,
sowie Formulierungshilfsstoffe enthält.

2. Schädlingsbekämpfungsmittel nach Anspruch 1, worin $R^1$ Wasserstoff oder Methyl, $R^2$ Methyl, $R^3$ und $R^4$ je Methyl und $R^5$ Wasserstoff bedeuten.

3. Schädlingsbekämpfungsmittel nach Anspruch 1, worin $R^1$ Wasserstoff und $R^2$ Methyl oder n-Propyl bedeuten.

4. Schädlingsbekämpfungsmittel nach Anspruch 1, dadurch gekennzeichnet, dass es eine wirksame Menge 2,2-Dimethyl-1,3-benzodioxan-8-yl-N-methylcarbamat sowie Formulierungshilfsstoffe enthält.

5. Schädlingsbekämpfungsmittel nach Anspruch 1, dadurch gekennzeichnet, dass es eine wirksame Menge mindestens einer Verbindung ausgewählt aus :

2-Aethyl-2-methyl-1,3-benzodioxan-8-yl-N-methylcarbamat,
2,2,4-Trimethyl-1,3-benzodioxan-8-yl-N-methylcarbamat,
2,2-Dimethyl-1,3-benzodioxan-8-yl-N-äthylcarbamat,
2,2-Dimethyl-1,3-benzodioxan-8-yl-N-(n-propyl)carbamat und
2,2-Diäthyl-1,3-benzodioxan-8-yl-N-methylcarbamat

sowie Formulierungshilfsstoffe enthält.

6. Schädlingsbekämpfungsmittel nach Anspruch 1, dadurch gekennzeichnet, dass es eine wirksame Menge, 2,2-Dimethyl-1,3-benzodioxan-8-N,N-dimethylcarbamat, sowie Formulierungshilfsstoffe enthält.

7. Verfahren zur Herstellung von Verbindungen der allgemeinen Formel

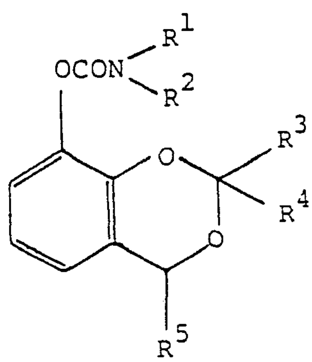

(I)

worin
$R^1$ Wasserstoff oder Methyl,
$R^2$, $R^3$ und $R^4$ unabhängig voneinander $C_{1-4}$-Alkyl und
$R^5$ Wasserstoff oder Methyl bedeuten,
dadurch gekennzeichnet, dass man

a) ein Phenol der allgemeinen Formel

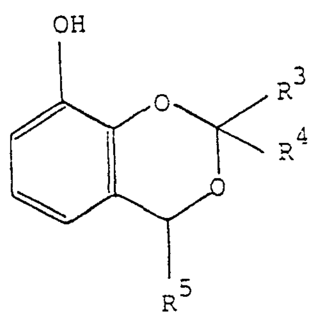

(II)

worin $R^3$, $R^4$ und $R^5$ die oben angegebenen Bedeutungen besitzen, mit einem Isocyanat der allgemeinen Formel

$$R^2N=C=O \quad \text{(III)}$$

worin $R^2$ die oben angegebene Bedeutung besitzt, umsetzt,

b) ein Phenol der allgemeinen Formel II, wie oben definiert, oder ein Alkalisalz davon, mit einem Carbamoylhalogenid der allgemeinen Formel

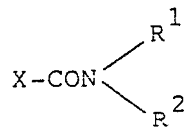

(IV)

worin $R^1$ und $R^2$ die oben angegebenen Bedeutungen besitzen und X ein Halogenatom bedeutet, umsetzt, oder

c) ein Phenol der allgemeinen Formel II, wie oben definiert, mit Phosgen und anschliessend einem Amin der allgemeinen Formel

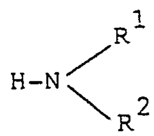

(V)

worin $R^1$ und $R^2$ die oben angegebenen Bedeutungen besitzen, umsetzt.

8. Verfahren zur Herstellung eines Schädlingsbekämpfungsmittels, dadurch gekennzeichnet, dass man mindestens eine der in den Ansprüchen 1 bis 6 genannten Verbindungen mit Formulierungshilfsstoffen vermischt.

9. Verfahren zur Bekämpfung von Schädlingen, dadurch gekennzeichnet, dass man die zu schützenden Gegenstände oder die Schädlinge selbst mit einer wirksamen Menge einer bzw. eines der in den Ansprüchen 1 bis 6 genannten Verbindungen bzw. Schädlingsbekämpfungsmittel behandelt.

10. Verwendung einer bzw. eines der in den Ansprüchen 1 bis 6 genannten Verbindungen bzw. Schädlingsbekämpfungsmittel zur Bekämpfung von Schädlingen.

**Claims** (for the Contracting States : BE, CH, DE, FR, GB, IT, LI, LU, NL, SE)

1. Compounds of the general formula

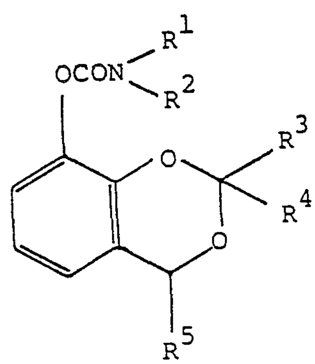

(I)

wherein

$R^1$ signifies hydrogen or methyl,

$R^2$, $R^3$ and $R^4$ each independently signify $C_{1-4}$-alkyl and

$R^5$ signifies hydrogen or methyl.

2. Compounds according to claim 1, wherein $R^1$ signifies hydrogen or methyl, $R^2$ signifies methyl, $R^3$ and $R^4$ each signify methyl and $R^5$ signifies hydrogen.

3. Compounds according to claim 1, wherein $R^1$ signifies hydrogen and $R^2$ signifies methyl or n-propyl.

4. 2,2-Dimethyl-1,3-benzodioxan-8-yl N-methylcarbamate.

5. A compound according to claim 1 selected from :

2-Ethyl-2-methyl-1,3-benzodioxan-8-yl N-methylcarbamate,
2,2,4-trimethyl-1,3-benzodioxan-8-yl N-methylcarbamate,
2,2-dimethyl-1,3-benzodioxan-8-yl N-ethylcarbamate,
2,2-dimethyl-1,3-benzodioxan-8-yl N-(n-propyl)carbamate and
2,2-diethyl-1,3-benzodioxan-8-yl N-methylcarbamate.

6. 2,2-Dimethyl-1,3-benzodioxan-8-yl N,N-dimethylcarbamate.

7. A compound according to claim 1 as a pest control agent.

8. A pest control composition, characterized in that it contains an effective amount of at least one compound of the general formula

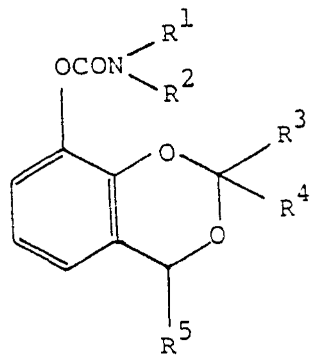

(I)

wherein
$R^1$ signifies hydrogen or methyl,
$R^2$, $R^3$ and $R^4$ each independently signify $C_{1-4}$-alkyl and
$R^5$ signifies hydrogen or methyl,
as well as formulation adjuvants.

9. A pest control composition according to claim 8, characterized in that it contains an effective amount of 2,2-dimethyl-1,3-benzodioxan-8-yl N-methylcarbamate as well as formulation adjuvants.

10. A process for the manufacture of compounds of the general formula

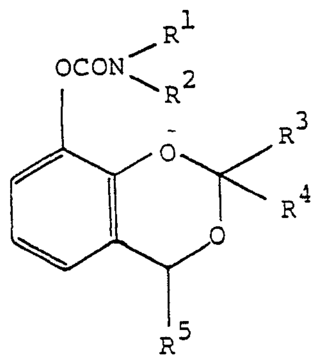

(I)

wherein
$R^1$ signifies hydrogen or methyl,
$R^2$, $R^3$ and $R^4$ each independently signify $C_{1-4}$-alkyl and
$R^5$ signifies hydrogen or methyl,
characterized by

a) reacting a phenol of the general formula

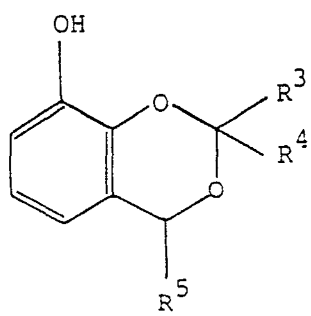

(II)

wherein $R^3$, $R^4$ and $R^5$ have the significances given above, with an isocyanate of the general formula

$$R^2N=C=O \qquad (III)$$

wherein $R^2$ has the significance given above,

b) reacting a phenol of general formula II, as defined above, or an alkali salt thereof with a carbamoyl halide of the general formula

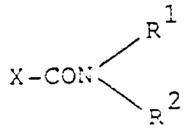

(IV)

wherein $R^1$ and $R^2$ have the significances given above and X signifies a halogen atom, or

c) reacting a phenol of general formula II, as defined above, with phosgene and subsequently an amine of the general formula

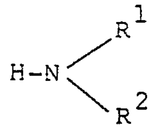

(V)

wherein $R^1$ and $R^2$ have the significances given above.

11. A method for the control of pests, characterized by treating the objects to be protected or the pests themselves with at least one of the compounds set forth in claims 1 to 6 or with a pest control composition set forth in claim 8 or 9.

12. The use of one of the compounds set forth in claims 1 to 6 or of a pest control composition set forth in claim 8 or 9 for the control of pests.

13. Compounds of the general formula

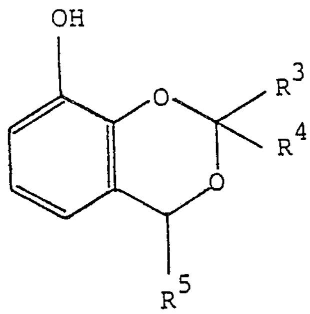

(II)

wherein

$R^3$ and $R^4$ each independently signify $C_{1-4}$-alkyl and

$R^5$ signifies hydrogen or methyl.

14. 2,2-Dimethyl-1,3-benzodioxan-8-ol.

15. Compounds of the general formula

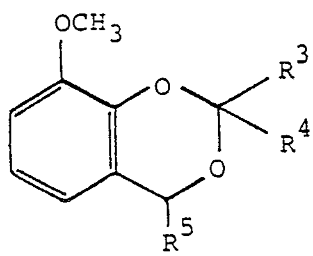

(VI)

wherein

$R^3$ and $R^4$ each independently signify $C_{1-4}$-alkyl and

$R^5$ signifies hydrogen or methyl.

16. 2,2-Dimethyl-8-methoxy-1,3-benzodioxan.

**Claims** (for the Contracting State AT)

1. A pest control composition, characterized in that it contains an effective amount of at least one compound of the general formula

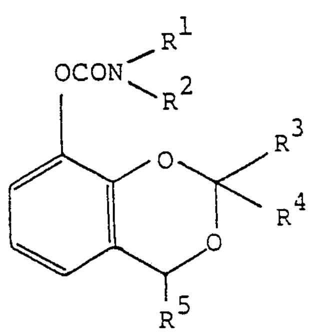

(I)

wherein
$R^1$ signifies hydrogen or methyl,
$R^2$, $R^3$ and $R^4$ each independently signify $C_{1-4}$-alkyl and
$R^5$ signifies hydrogen or methyl,
as well as formulation adjuvants.

2. A pest control composition according to claim 1, wherein $R^1$ signifies hydrogen or methyl, $R^2$ signifies methyl, $R^3$ and $R^4$ each signify methyl and $R^5$ signifies hydrogen.

3. A pest control composition according to claim 1, wherein $R^1$ signifies hydrogen and $R^2$ signifies methyl or n-propyl.

4. A pest control composition according to claim 1, characterized in that it contains an effective amount of 2,2-dimethyl-1,3-benzodioxan-8-yl N-methylcarbamate as well as formulation adjuvants.

5. A pest control composition according to claim 1, characterized in that it contains an effective amount of at least one compound selected from :

2-Ethyl-2-methyl-1,3-benzodioxan-8-yl N-methylcarbamate,
2,2,4-trimethyl-1,3-benzodioxan-8-yl N-methylcarbamate,
2,2-dimethyl-1,3-benzodioxan-8-yl N-ethylcarbamate,
2,2-dimethyl-1,3-benzodioxan-8-yl N-(n-propyl)carbamate and
2,2-diethyl-1,3-benzodioxan-8-yl N-methylcarbamate

as well as formulation adjuvants.

6. A pest control composition according to claim 1, characterized in that it contains an effective amount of 2,2-dimethyl-1,3-benzodioxan-8-yl N,N-dimethylcarbamate as well as formulation adjuvants.

7. A process for the manufacture of compounds of the general formula

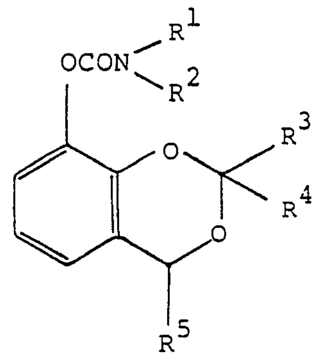

(I)

wherein
$R^1$ signifies hydrogen or methyl,
$R^2$, $R^3$ and $R^4$ each independently signify $C_{1-4}$-alkyl and
$R^5$ signifies hydrogen or methyl,
characterized by

a) reacting a phenol of the general formula

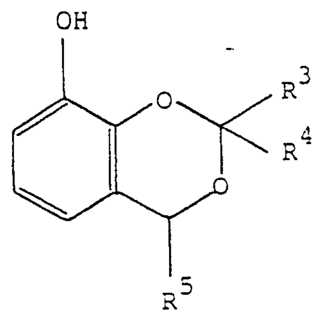

(II)

wherein $R^3$, $R^4$ and $R^5$ have the significances given above, with an isocyanate of the general formula

$$R^2N{=}C{=}O \quad (III)$$

wherein $R^2$ has the significance given above,

b) reacting a phenol of general formula II, as defined above, or an alkali salt thereof with a carbamoyl halide of the general formula

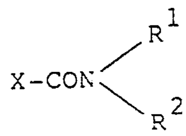

(IV)

wherein $R^1$ and $R^2$ have the significances given above and X signifies a halogen atom, or

c) reacting a phenol of general formula II as defined above, with phosgene and subsequently an amine of the general formula

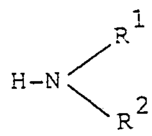

wherein $R^1$ and $R^2$ have the significances given above.

8. A process for the manufacture of a pest control composition, characterized by mixing at least one of the compounds set forth in claims 1 to 6 with formulation adjuvants.

9. A method for the control of pests, characterized by treating the objects to be protected or the pests themselves with an effective amount of one of the compounds or pest control compositions set forth in claims 1 to 6.

10. The use of one of the compounds or pest control compositions set forth in claims 1 to 6 for the control of pests.

**Revendications** (pour les Etats contractants : BE, CH, DE, FR, GB, IT, LI, LU, NL, SE)

1. Composés de formule générale :

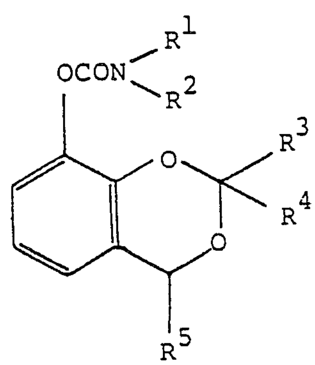

(I)

dans laquelle :

$R^1$ est l'hydrogène ou le méthyle ;

chaque $R^2$, $R^3$ et $R^4$ indépendamment l'un de l'autre est un alkyle en $C_1$-$C_4$ ; et

$R^5$ est l'hydrogène ou le méthyle.

2. Composés selon la revendication 1, dans lesquels $R^1$ est l'hydrogène ou le méthyle, $R^2$ est le méthyle, $R^3$ et $R^4$ sont tous deux des méthyle et $R^5$ est l'hydrogène.

3. Composés selon la revendication 1, dans lesquels $R^1$ est l'hydrogène et $R^2$ est le méthyle ou le n-propyle.

4. 2,2-diméthyl-1,3-benzodioxane-8-yl-N-méthylcarbamate.

5. Un composé selon la revendication 1, choisi parmi :

2-éthyl-2-méthyl-1,3-benzodioxane-8-yl-N-méthylcarbamate ;
2,2,4-triméthyl-1,3-benzodioxane-8-yl-N-méthylcarbamate ;
2,2-diméthyl-1,3-benzodioxane-8-yl-N-éthylcarbamate ;
2,2-diméthyl-1,3-benzodioxane-8-yl-N-(n-propyl)-carbamate et
2,2-diéthyl-1,3-benzodioxane-8-yl-N-méthylcarbamate.

6. 2,2-diméthyl-1,3-benzodioxane-8-yl-N,N-diméthylcarbamate.

7. Composés selon la revendication 1 en tant qu'agent de lutte antiparasitaire.

8. Agent de lutte antiparasitaire, caractérisé en ce qu'il contient une quantité efficace d'au moins un composé de formule générale :

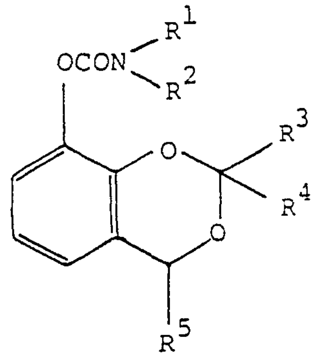

(I)

dans laquelle :

$R^1$ est l'hydrogène ou le méthyle ;

chaque $R^2$, $R^3$ et $R^4$ indépendamment l'un de l'autre est un alkyle en $C_1$-$C_4$ ; et

$R^5$ est l'hydrogène ou le méthyle ;

ainsi que des produits auxiliaires de formulation.

9. Agent de lutte antiparasitaire selon la revendication 8, caractérisé en ce qu'il contient une quantité efficace de 2,2-diméthyl-1,3-benzodioxane-8-yl-N-méthylcarbamate ainsi que des produits auxiliaires de formulation.

10. Procédé de préparation de composés de formule générale I :

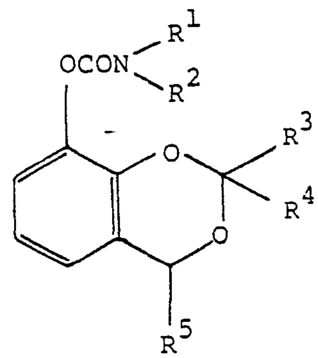

(I)

dans laquelle :

$R^1$ est l'hydrogène ou le méthyle ;

chaque $R^2$, $R^3$ et $R^4$ indépendamment l'un de l'autre est un alkyle en $C_1$-$C_4$ ; et

$R^5$ est l'hydrogène ou le méthyle ;

caractérisé en ce que :

a) on condense un phénol de formule générale :

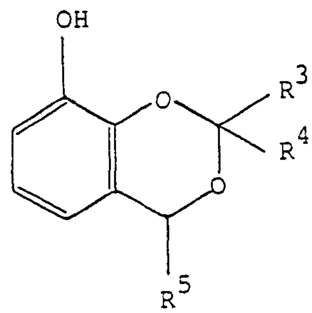

(II)

dans laquelle $R^3$, $R^4$ et $R^5$ possèdent les significations indiquées ci-dessus ; avec un isocyanate de formule générale :

$$R^2N=C=O \qquad (III)$$

dans laquelle $R^3$ possède la signification indiquée ci-dessus ;

b) on condense un phénol de formule générale II, comme défini ci-dessus, ou un de ses sels alcalins, avec un halogénure de carbamoyle de formule générale :

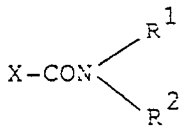

(IV)

dans laquelle $R^1$ et $R^2$ possèdent les significations indiquées ci-dessus et X est un atome d'halogène ; ou

c) on condense un phénol de formule générale II, comme défini ci-dessus, avec du phosgène et ensuite une amine de formule générale :

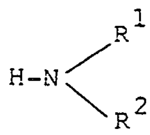

(V)

dans laquelle $R^1$ et $R^2$ possèdent les significations indiquées ci-dessus.

11. Procédé de lutte contre des parasites, caractérisé en ce qu'on traite les objets à protéger ou les parasites eux-mêmes avec au moins l'un des composés cité dans les revendications 1 à 6 ou avec un agent de lutte antiparasitaire cité dans la revendication 8 ou 9.

12. Utilisation d'un des composés cités dans les revendications 1 à 6 ou d'un agent de lutte antiparasitaire cité dans la revendication 8 ou 9 pour combattre des parasites.

13. Composés de formule générale :

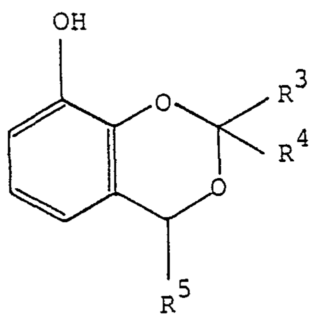

(II)

dans laquelle :

chaque $R^3$ et $R^4$ indépendamment l'un de l'autre est un alkyle en $C_1$-$C_4$ ; et

$R^5$ est l'hydrogène ou le méthyle.

14. 2,2-diméthyl-1,3-benzodioxane-8-ol.

15. Composés de formule générale :

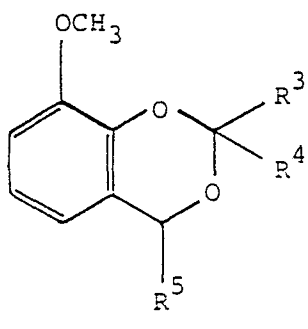

(VI)

dans laquelle :

chaque $R^3$ et $R^4$ indépendamment l'un de l'autre est un alkyle en $C_1$-$C_4$ ; et

$R^5$ est l'hydrogène ou le méthyle.

16. 2,2-diméthyl-8-méthoxy-1,3-benzodioxane.

**Revendications** (pour l'Etat contractant AT)

1. Agent de lutte antiparasitaire, caractérisé en ce qu'il contient une quantité efficace d'au moins un composé de formule générale :

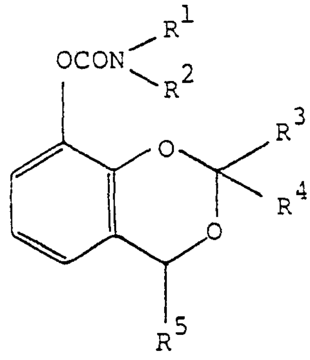

(I)

dans laquelle :

$R^1$ est l'hydrogène ou le méthyle ;

chaque $R^2$, $R^3$ et $R^4$ indépendamment l'un de l'autre est un alkyle en $C_1$-$C_4$ ; et

$R^5$ est l'hydrogène ou le méthyle,

ainsi que des produits auxiliaires de formulation.

2. Agent de lutte antiparasitaire selon la revendication 1, dans lequel $R^1$ est l'hydrogène ou le méthyle, $R^2$ le méthyle, $R^3$ et $R^4$ sont tous deux des méthyle et $R^5$ est l'hydrogène.

3. Agent de lutte antiparasitaire selon la revendication 1, dans lequel $R^1$ est l'hydrogène et $R^2$ est le méthyle ou le n-propyle.

4. Agent de lutte antiparasitaire selon la revendication 1, caractérisé en ce qu'il contient une quantité efficace de 2,2-diméthyl-1,3-benzodioxane-8-yl-N-méthylcarbamate ainsi que des produits auxiliaires de formulation.

5. Agent de lutte antiparasitaire selon la revendication 1, caractérisé en ce qu'il contient une quantité efficace d'au moins un composé choisi parmi :

2-éthyl-2-méthyl-1,3-benzodioxane-8-yl-N-méthylcarbamate,
2,2,4-triméthyl-1,3-benzodioxane-8-yl-N-méthylcarbamate,
2,2-diméthyl-1,3-benzodioxane-8-yl-N-éthylcarbamate,
2,2-diméthyl-1,3-benzodioxane-8-yl-N-(n-propyl)-carbamate, et
2,2-diéthyl-1,3-benzodioxane-8-yl-N-méthylcarbamate

ainsi que des produits auxiliaires de formulation.

6. Agent de lutte antiparasitaire selon la revendication 1, caractérisé en ce qu'il contient une quantité efficace de 2,2-diméthyl-1,3-benzodioxane-8-yl-N,N-diméthylcarbamate, ainsi que des produits auxiliaires de formulation.

7. Procédé de préparation de composés de formule générale

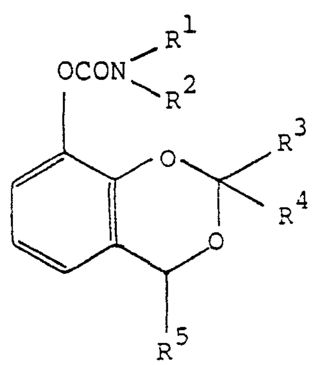

(I)

dans laquelle :

$R^1$ est l'hydrogène ou le méthyle ;

chaque $R^2$, $R^3$ et $R^4$ indépendamment l'un de l'autre est un alkyle en $C_1$-$C_4$ ; et

$R^5$ est l'hydrogène ou le méthyle ;

caractérisé en ce que :

a) on condense un phénol de formule générale :

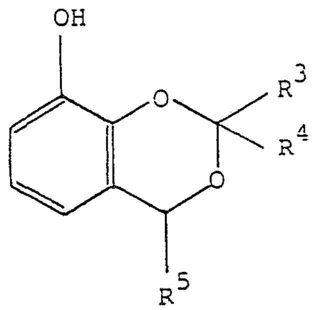

(II)

dans laquelle $R^3$, $R^4$ et $R^5$ possèdent les significations indiquées ci-dessus ; avec un isocyanate de formule générale :

$$R^2N=C=O \quad (III)$$

dans laquelle $R^2$ possède la signification indiquée ci-dessus ;

b) on condense un phénol de formule générale II, comme défini ci-dessus, ou un de ses sels alcalins, avec un halogénure de carbamoyle de formule générale :

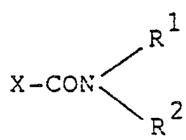

(IV)

dans laquelle $R^1$ et $R^2$ possèdent les significations indiquées ci-dessus et X est un atome d'halogène ; ou

c) on condense un phénol de formule générale II, comme défini ci-dessus, avec du phosgène et ensuite une amine de formule générale :

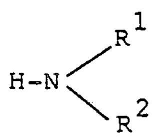

(V)

dans laquelle $R^1$ et $R^2$ possèdent les significations indiquées ci-dessus.

8. Procédé de fabrication d'un agent de lutte antiparasitaire, caractérisé en ce qu'on mélange au moins l'un des composés cité dans les revendications 1 à 6 avec des produits auxiliaires de formulation.

9. Procédé de lutte contre des parasites, caractérisé en ce que l'on traite les objets à protéger ou les parasites eux-mêmes avec une quantité efficace d'un des composés ou d'un des agents de lutte antiparasitaire cités dans les revendications 1 à 6.

10. Utilisation d'un des composés ou d'un des agents de lutte antiparasitaire cités dans les revendications 1 à 6 pour combattre des parasites.